# EUROPEAN PATENT SPECIFICATION

(11) **EP 1 825 254 B1**
(45) Date of publication and mention of the grant of the patent: **02.04.2014**
(21) Application number: 05851634.5
(22) Date of filing: 15.11.2005
(51) Int. Cl.: G01N 33/68

(54) **METHOD FOR OPTIMIZING THIOPURINE EFFICACY AND TOXICITY USING MASS SPECTROMETRY**
VERFAHREN ZUR OPTIMIERUNG DER THIOPURINWIRKUNG UND -TOXIZITÄT MITHILFE VON MASSENSPEKTROMETRIE
PROCÉDÉ POUR OPTIMISER L'EFFICACITÉ DE LA THIOPURINE ET RÉDUIRE LA TOXICITÉ AU MOYEN DE LA SPECTROMÉTRIE DE MASSE

(30) Priority: 16.11.2004 US 628728 P; 14.11.2005 US 274104
(43) Date of publication of application: 29.08.2007
(73) Proprietor: Nestec S.A., 1800 Vevey (CH)
(72) Inventor: DERVIEUX, Thierry, Encino, CA 91316 (US)
(74) Representative: Rupp, Christian
(86) International application number: PCT/US2005/041272
(87) International publication number: WO 2006/055527

(56) References cited:
- US-A1- 2001 006 970
- US-A1- 2002 028 476
- US-B2- 6 680 302
- DUBINSKY ET AL: "6-MP metabolite profiles provide a biochemical explanation for 6-MP resistance in patients with inflammatory bowel disease" GASTROENTEROLOGY, ELSEVIER, PHILADELPHIA, PA, vol. 122, no. 4, 1 April 2002 (2002-04-01), pages 904-915, XP005874191 ISSN: 0016-5085
- CUFFARI C ET AL: "UTILISATION OF ERYTHROCYTE 6-THIOGUANINE METABOLITE LEVELS TO OPTIMISE AZATHIOPRINE THERAPY IN PATIENTS WITH INFLAMMATORY BOWEL DISEASE" GUT, BRITISH MEDICAL ASSOCIATION, LONDON, vol. 48, no. 5, 1 May 2001 (2001-05-01), pages 642-646, XP009049769 ISSN: 0017-5749
- LOUIS EDOUARD ET AL: "Optimizing treatment with thioguanine derivatives in inflammatory bowel disease." BEST PRACTICE & RESEARCH. CLINICAL GASTROENTEROLOGY FEB 2003, vol. 17, no. 1, February 2003 (2003-02), pages 37-46, XP002505472 ISSN: 1521-6918
- DERVIEUX T ET AL: "Identification of 6-methylmercaptopurine derivative formed during acid hydrolysis of thiopurine nucleotides in erythrocytes, using liquid chromatogrphy-mass spectrometry" CLINICAL CHEMISTRY, AMERICAN ASSOCIATION FOR CLINICAL CHEMISTRY, WASHINGTON, DC, vol. 44, no. 12, 1 December 1998 (1998-12-01), pages 2511-2515, XP003000323 ISSN: 0009-9147
- DERVIEUX T. ET AL.: 'Identification of 6-methylmercaptopurine derivative formed during acid hydrolysis of thiopurine nucleotides in erythrocytes, using liquid chromatogrphy-mass spectrometry' CLIN. CHEM vol. 44, no. 12, 01 December 1998, pages 2511 - 2515, XP003000323

## Description

### BACKGROUND OF THE INVENTION

Thiopurines (mercaptopurine and azathioprine) were developed in the 1950's by James Black, Gertnude Elion, and George Hitchings and are widely used as immunosuppressants in the treatment of inflammatory bowel disease (Dubinsky, M.C. et al., Gastroenterology 122:904-915 (2002)).

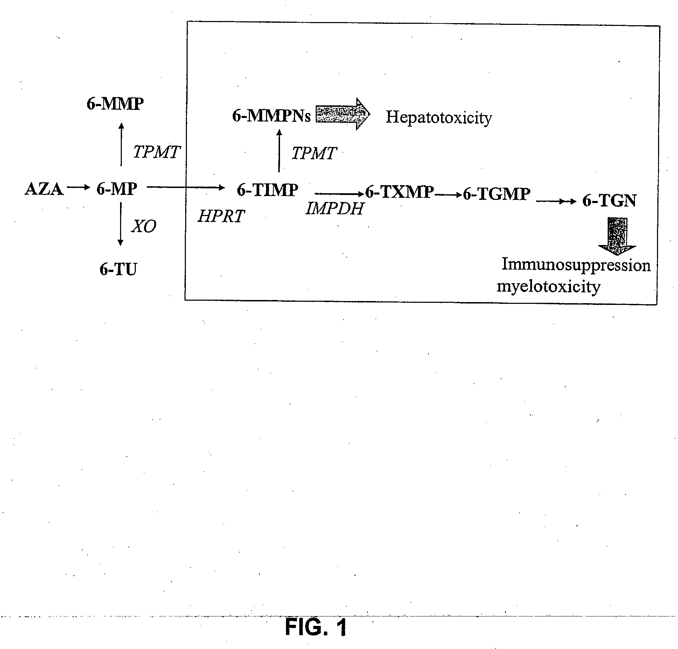

Mercaptopurine, the analogue of hypoxanthine is activated intracellularly to thioinosine monophosphate (6-TIMP) by hypoxanthine phosphoribosyltransferase which is further converted to thioguanine nucleotides (TGNs) by a multistep process implicating inosine monophosphate dehydrogenase and guanosine monophosphate synthase (Lennard, L. Eun. J. Clin. Pharmacol. 43:329-339 (1992)). This anabolic route is in competition with methylation of 6-TIMP into methylmercaptopurine nucleotides (6-MMPN) by polymorphic thiopurine methyltransferase (TPMT). The intracellular activation of mercaptopurine is also in competition with two extracellular catabolic routes, i) oxidation of mercaptopurine to thiouric acid by xanthine oxidase, and ii) methylation to 6-methylmercaptopurine by TPMT (**Figure 1**). The antiproliferative effects of thiopurines are associated with incorporation of deoxythioguanine nucleotides into the genomic DNA and *de novo* purine synthesis inhibition by TGNs and 6-MMPNs (Dervieux, T. et al., Cancer Res 61:5810-5816 (2001)). In contrast, the immunosuppressive effects of azathioprine appear to be associated with modulation of RAC-1 activity by thioguanosine triphosphate (TGTP) (Tiede, I. et al., J Clin Invest. 111: 1133-1145 (2003)),

Pioneer work by Weinshilboum and Sladek in the late 70's demonstrated that TPMT activity was polymorphic and transmitted as an autosomal co-dominant trait (Weinshilboum, R. M. et al., Am. J. Hum. Gent. 32:651-662 (1980)). About 1 in 300 individuals cannot produce functional TPMT enzyme; 10% of the population is Heterozygous for this polymorphism, and have intermediate levels of TPMT activity. The remaining 90% carry two wild type alleles and have full TPMT activity. The molecular basis for TPMT deficiency was discovered by Evans and co-workers and is well established (Tai, H.L. et al., An. J. Hum. Genet. 58:694-702 (1996); Yates, C.R. et al., Ann. Intern. Med. 126:608-614 (1997)). Three non-synonymous SNPs account for over 90% of the clinically relevant TPMT mutations (Tai, H.L. et al., Am. J. Hum. Genet. 58:694-702 (1996); Yates, C.R. et al., Ann. Intern. Med. 126:608-614 (1997); Otterness, D. et al., Clin. Pharmacol. Ther. 62:60-73 (1997)) and the resulting amino acid substitutions do not affect the levels of TPMT transcript, but render the protein more susceptible to destruction through ubiquitination.(Tai, H.L. et al., Proc. Natl. Acad. Sci. U.S.A. 94:6444-6449 (1997); Tai, H.-L. et al., Pharmacogenetics 9:641-650. (1999)).

Mercaptopurine and azathioprine therapy can result in myelosuppression. Patients with the homozygous mutant TPMT genotype can experience life-threatening myelosuppression due to the extensive shunt of the metabolism towards formation of TGNs, and can require dosage reductions of up to ten-fold in order to tolerate therapy (Lennard, L. et al., Lancet 336:225-229 (1990); Lennard, L. et al., Arch. Dis. Child 69:577-579 (1993)). More commonly, patient carriers of heterozygous mutations require dosage reductions of 10% to 50% (Relling, M. V. et al., J. Natl. Cancer Inst. 91:2001-2008 (1999); Dervieux, T. et al., Leukemia. (2001)), but can be also at risk of severe myelosuppression (Tassaneeyakul, W. et al., Transplantation 76:265-266 (2003)). In contrast, patient with a wild type TPMT genotype are less likely to accumulate TGN concentration in the toxic range, but are more likely to accumulate potentially hepatotoxic 6-MMPN during dose escalation (Dubinsky, M.C. et al., Gastroenterology 122:904-915 (2002); Schutz, E. et al., Lancet 341:436 (1993); Oh, K. T. et al., Rheumatology (Oxford) 43:156-163 (2004)).

A value of a therapeutic drug monitoring of azathioprine therapy with erythrocyte TGNs and 6-MMPNs is established, and therapeutic thresholds associated with increased likelihood to efficacy, increase risk for leucopenia, and increased risk for hepatotoxicity can help adjust azathioprine dosage to maintain remission in patients with inflammatory bowel disease (Dubinsky, M.C. et al., Gastroenterology 122:904-915 (2002); Oh, K. T. et al., Rheumatology (Oxford) 43:156-163 (2004); Marra, C. A. et al. J Rheumatol 29:2507-2512 (2002); Dubinsky, M. C. et al., Gastroenterology 118:705-713 (2000)).

Chromatographic methods for the determination of intracellular thiopurine nucleotide concentrations in red blood cells (Cuffari, C. et al., Clin Gastroenterol Hepatol 2:410-417 (2004); Cuffari, C, et al., Gut 39:401-406 (1996)1 Lavi, L. E. et al., Anal. Biochem. 144:514-521 (1985); Erdmann, G. R. et al., Biomed. Chromatogr. 4:47-51 (1990)) or peripheral lymphoblasts (Lennard, L, et al., J. Chromatogr. 583:83-90 (1992); Dervieux, T. et al., Clin. Chem 44:551-555 (1998); Keuzenkamp-Jansen, C. W. et al., J. Chromatogr. B. Biomed. Appl. 672:53-61 (1995); Zimmerman, T, P. et al., Cancer Res. 34:221-224 (1974); Erdmann, G. R. et al., J. Chromatogr. 571:149-156 (1991)) have been developed. Many of these methods are based on acid hydrolysis of the thiopurine nucleotide moieties to thiopurine bases (Cuffari, C, et al., Gut 39:401-406 (1996); Lavi, L. E. et al., Anal. Biochem. 144:514-521 (1985); Erdmann, G. R. et al., Biomed. Chromatogr. 4:47-51 (1990); Keuzenkamp-Jansen, C. W. et al., J. Chromatogr. B. Biomed. Appl. 672:53-61 (1995)) and use ultraviolet detection (Lavi, L. E. et al., Anal. Biochem. 144:514-521 (1985); Erdmann, G. R. et al., Biomed. Chromatogr. 4:47-51 (1990)) or fluorometric detection (Cuffari, C, et al., Gut 39:401-406 (1996); Keuzenkamp-Jansen, C. W. et, al., J. Chromatogr. B. Biomed. Appl. 672:53-61 (1995)).

Assays for the quantification of 6-TGN and 6-MMPN became commercially available in 1999 based on the findings that 6-TGN and 6-MMPN levels were associated with efficacy and toxicity to azathioprine in patients with inflammatory bowel disease (Oh, K. T. et al., Rheumatology (Oxford) 43:156-163 (2004)). The initial sample treatment procedure used the method developed by Lynne Lennard in Sheffield (Lavi, L. E. et al., Anal. Biochem. 144:514-521 (1985)) and modified by Yves Theoret at St Justine Hospital (Dubinsky, M. C. et al., Gastroenterology 118:705-713 (2000)) to established therapeutic threshold of 6-TGNs associated with efficacy (>235 pmol/8x10⁸ cells), leukopenia (>400 pmol/8x10⁸ cells), and 6-MMPN associated with hepatotoxicity (>5700 pmol/8x10⁸ cells). To improve throughput and avoid toxic mercury waste generated, the sample treatment procedure was changed to the Dervieux and Boulieu's method. Equivalency between both analytical procedures was established using patients samples under azathioprine therapy. There was a good correlation between methods (R²>0.90) with an analytical bias of 16% higher TGNs and 2% lower 6-MMPN in the Dervieux-Boulieu method compared to the Lennard method. However, other laboratories have found significant 1.4-fold (Dervieux, T. et al., Clin Chem, In press. (2001)) to 2.6 fold (Dervieux, T, et al. Clin. Chem. 44:2511-2515 (1998)) differences in TGN concentrations between these two methods. This illustrates the need to standardize the analytical procedures for the determination of thiopurine nucleotides, as emphasized by Armstrong and Oellerich's group (Dervieux, T, et al. Clin. Chem. 44:2511-2515 (1998); Stefan, C. et al., Clin Biochem JID - 0133660 37:764-771 (2004)).

Several studies have demonstrated the cost effectiveness of screening for TPMT polymorphisms (Shipkova, M. et al., Clin Chem 49:260-268 (2003); Armstrong, V. W. et al., Ther Drug Monit 26:220-226 (2004)) and data from clinical trials have established the value of measuring 6-TGN and 6-MMPNs to optimize therapy (Dubinsky, M.C. et al., Gastroenterology 122:904-915 (2002); Oh, K. T. et al., Rheumatology (Oxford) 43:156-163 (2004); Marra, C. A. et al. J Rheumatol 29:2507-2512 (2002); Dubinsky, M. C. et al., Gastroenterology 118:705-713 (2000)):

U.S. Patent No. 6,680,302 ("the'302 patent"), which is incorporated herein by reference, discloses methods for optimizing therapeutic efficacy of 6-mercaptopurine drug treatment by determining levels of various metabolites, such as 6-thioguanine nucleotide and 6-methyl-mercaptopurine nucleotide. The level of 6-thioguanine can be determined, for example, in red blood cells using high pressure liquid chromatography.

Despite the advances of the '302 patent, there remains a need in the art for more advanced methods for determining the concentration levels of 6-thioguanine nucleotide (6-TGN) and methyl 6-mercaptopurine nucleotide (6-MMPN), to thereby optimize purine drug therapy. The present invention fulfills these and other needs.

US 2001/006970 discloses a method for optimizing therapeutic efficacy in a subject receiving a drug providing 6-hioguanine nucleotide (6-TGN), said method comprising: (a) determining a concentration level of 6-TGN in said subject using methods well known in the art including, for example high pressure liquid chromatography (HPLC); and (b) increasing the subsequent dose of said drug when said concentration level of 6-TGN is less than a predetermined 6-TGN efficacy value.

### SUMMARY OF THE INVENTION

### Embodiments of the invention:

1) A method for optimizing therapeutic efficacy in a subject receiving a drug providing 6-thioguanine nucleotide (6-TGN), said method comprising:
   (a) determining a concentration level of 6-TGN in said subject using an analytical technique having an ionizing source, wherein said analytical technique is a mass spectrometer;
   (b) determining a concentration level of 6-methyl-mercaptopurine nucleotide (6- MMPN) in said subject using an analytical technique having an ionizing source, wherein said analytical technique is multiple reaction monitoring using a mass spectrometer, wherein determination of 6-TGN and 6-MMPN concentration levels occur simultaneously; and
   (c) recommending increasing the subsequent dose of said drug when said concentration level of 6-TGN is less than a predetermined 6-TON efficacy value; or
   (d) recommending decreasing the subsequent dose of said drug when said concentration level of 6-MMPN is greater than a predetermined 6-MMPN toxicity value.
2) A method for reducing toxicity in a subject receiving a drug providing 6- thioguanine nucleotide (6-TGN), said method comprising:
   (a) determining a concentration level of 6-TON in said subject using an analytical technique having an ionizing source;
   (b) determining a concentration level of 6-methyl-me rcaptopurine nucleotide (6-MMPN) in said subject using an analytical technique having an ionizing source, wherein said analytical technique is multiple reaction monitoring using a mass spectrometer, wherein determination of 6-TGN and 6-MMPN concentration levels occur simultaneously; and (c) recommending decreasing the subsequent dose of said drug when said concentration level of 6-TON is greater than a predetermined 6-TGN toxicity value; or
   (d) recommending decreasing the subsequent dose of said drug when said concentration level of 6-MMPN is greater than a predetermined 6-MMPN toxicity value.
3) A method for optimizing therapeutic efficacy for treatment of an immune- mediated gastrointestinal disorder, said method comprising:
   determining a concentration level using an analytical technique having an ionizing source wherein said analytical technique is multiple reaction monitoring using a mass spectrometer, of 6-thioguanine nucleotide (6-TGN) and 6-methyl-mercaptopurine nucleotide (6-MMPN) in a subject administered a drug providing 6-TGN, said subject having said immune-mediated gastrointestinal disorder, wherein determination of 6-TGN and 6-MMPN concentration levels occur simultaneously, wherein a concentration level of 6-TGN less than a predetermined 6-TGN efficacy value indicates recommending an increase in the amount of said drug subsequently administered to said subject, and
   wherein a concentration level of 6-TGN greater than a predetermined 6-TGN toxicity value, or a concentration level of 6-MMPN greater than a predetermined 6-MMPN toxicity value indicates recommending a decrease in the amount of said drug subsequently administered to said subject.
4) The method of any one of embodiments 1 to 2, wherein said subject has a disease or disorder selected from the group consisting of an immune-mediated gastrointestinal disorder, an autoimmune disease, and graft versus host disease.
5) The method of any one of embodiments 1 to 4, wherein said drug is selected from the group consisting of 6-mercaptopurine, azathioprine, 6-thioguanine, and 6-methylmercaptopurine riboside.
6) The method of any one of embodiments 1 to 5, wherein said ionizing source is selected from a group consisting of an electrospray ion source, an atmospheric pressure ionization source, and a matrix assisted laser desorption ion source.
7) The method of any one of embodiments 1 to 6, wherein said analytical technique further comprises liquid chromatography (LC) separation.
8) The method of embodiment 7, wherein said concentration level of 6-thioguanine nucleotide (6-TGN) or 6-methyl-mercaptopurine nucleotide (6-MMPN) is determined using LC-tandem mass spectroscopy.
9) The method of any one of embodiments 1, 2 and 3 to 8, wherein a transition of a mass-to-charge ratio (m/z) of 168-151 is used to identify 6-TGN.
10) The method of any one of claims 1-9, wherein the mass to charge ratio (m/z) of 158-110 transition is used to identify 6-MMPN.
11) The method of any one of embodiments 1, 3-10 wherein said predetermined 6-TGN efficacy value is selected from the group consisting of 200, 210, 220, 230, 235, 240, 250, 10 260, 280, 300 and 350 pmol per 8x10⁸ red blood cells.
12) The method of claim 11, wherein said predetermined 6-TGN efficacy value is 235 pmol per 8x10⁸ red blood cells.
13) The method of any one of embodiments 2-12, wherein said predetermined 6-TGN toxicity value is selected from the group consisting of 350, 370, 390, 400, 410, 425, and 450 pmol per 8x10⁸ red blood cells.
14) The method of embodiment 13, wherein said predetermined 6-TGN toxicity value is 400 pmol per 8x10⁸red blood cells.
15) The method of any one of embodiments 1-14, wherein said predetermined 6-MMPN toxicity value is selected from the group consisting of 5500, 5600, 5700, 6000, 6500, 7000, 20 7500, and 8000 pmol per 8x10⁸ red blood cells.
16) The method of embodiment 15, wherein said predetermined 6-MMPN toxicity value is 5700 pmol per 8x10⁸ red blood cells.
17) The method of any one of embodiments 1-16, where a testing laboratory provides dosing instructions for said drug selected from the group consisting of a package insert to accompany said drug, a guideline for using said drug, a lab results with preferred drug doses, a data sheet, and a look-up table setting forth preferred drug doses.

### BRIEF DESCRIPTION OF THE DRAWINGS

**Figure 1****:** is a schematic which illustrates azathioprine metabolism. The abbreviations are as follows: AZA: azathioprine, IMPDH: inosine monophosphate dehydrogenase; XO: xanthine oxidase; 6-TU: thiouric acid; TIMP: Thioinosine monophosphate; TXMP: thioxanthosine monophosphate; and TGMP: thioguanosine monophosphate

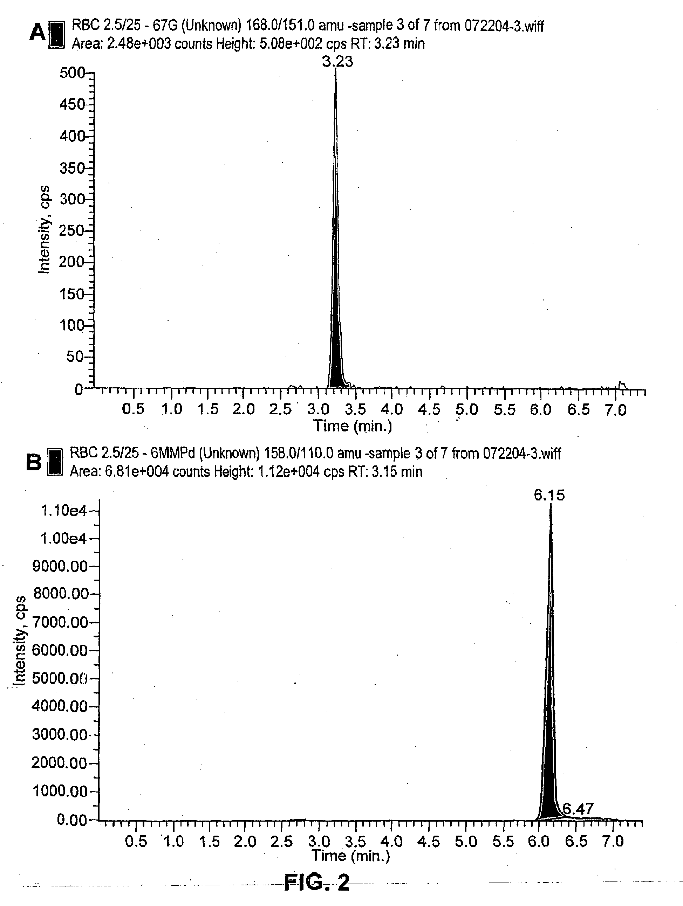

**Figure 2****:** illustrates one embodiment of a thiopurine erythrocyte LC/MS/MS chromatogram. A representative RBC standard spiked with 2.5 µM 6-TG and 25.0 µM 6-MMP and subjected to the sample treatment procedure is presented. Multiple reaction monitoring consisted of a m/z: 168→151 transition for 6-TG (panel A: retention time: 3.2 minutes) and m/z:158→110 for 6-MMP derivative (panel B: retention time: 6.1 minutes).

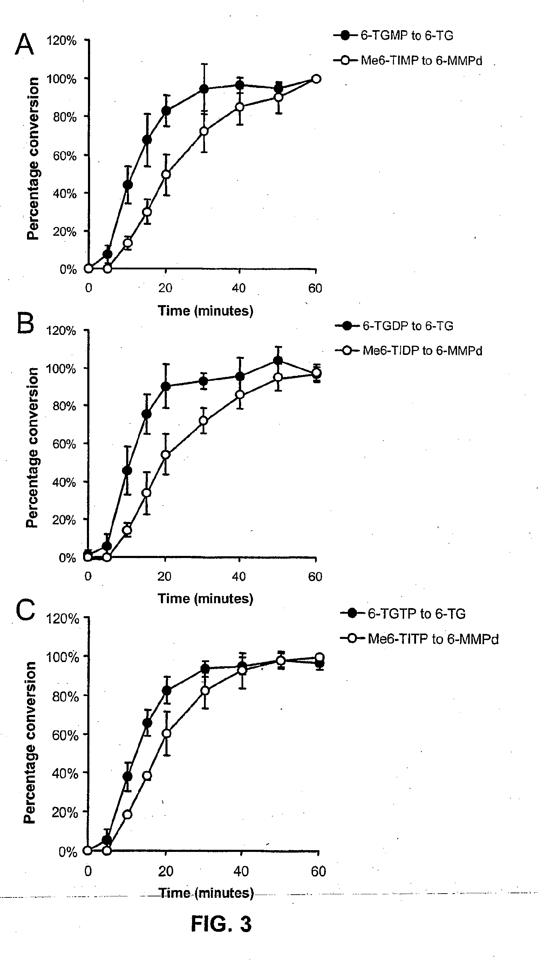

**Figure 3****:** illustrates kinetics of conversion of thiopurine nucleotides during acid hydrolysis. 6-thioguanine and 6-methylmercaptopurine nucleoside mono (panel A), di (panel B), and triphosphate (panel C) were spiked in RBC hemolysates at final concentrations of 10µM (6-TGNs) and 100µM (6-MMPNs). Samples were subjected to the deproteinization step with perchloric acid in presence of DTT. Acid extracts were subsequently heated to convert 6-TGNs to 6-TG and 6-MMPNs to the derivative. Note that the kinetics of hydrolysis of TGNs (6-TGMP, 6-TGDP, 6-TGTP) to 6-TG was faster than that of 6-MMPNs (MeTIMP, MeTIDP, MeTITP) to the derivative.

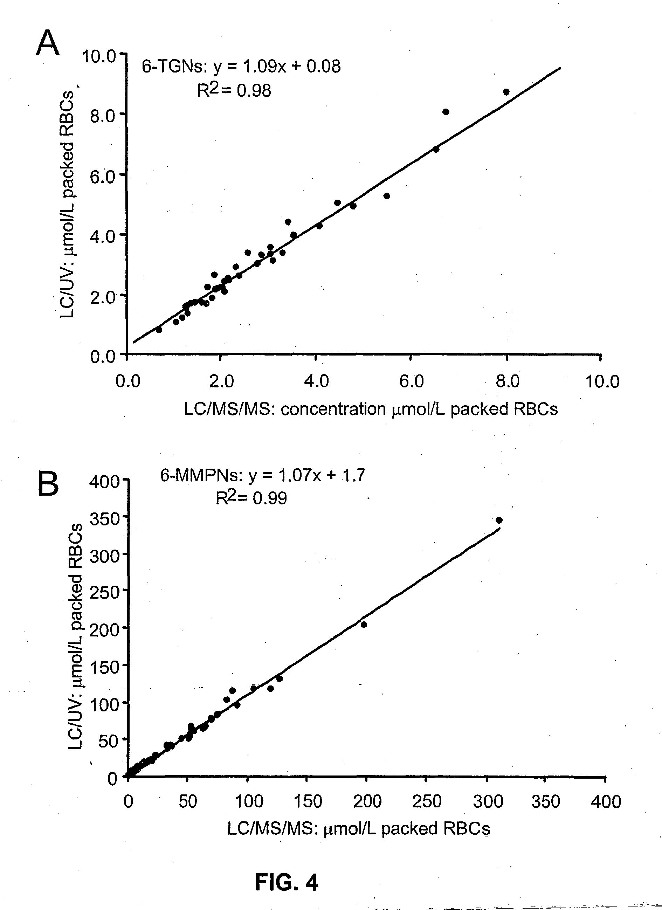

**Figure 4****:** illustrates a method comparison. RBC 6-TGNs and 6-MMPNs from patients under thiopurine therapy were measured using the conventional HPLC/LUV method (Erdmann, G. R. et al., Biomed. Chromatogr. 4:47-51 (1990)) and compared to the LC/MS/MS method (panel A: TGN concentrations; panel B: 6-MMPN concentrations). Regression coefficient with slopes and intercept are given.

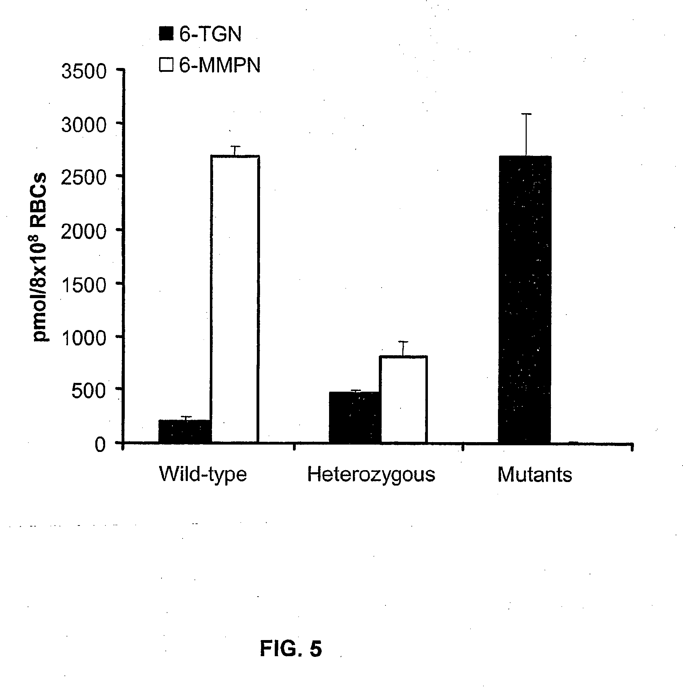

**Figure 5****:** illustrates erythrocyte 6-TGNs and 6-MMPNs levels by TPMT genotype. RBC 6-TGNs and 6-MMPNs were measured in 1523 patients under azathioprine or mercaptopurine therapy. Patient carriers of the heterozygous genotype presented higher 6-TGN concentrations compared to those carriers of the homozygous wild type genotype (p<0.0001). Those carriers of the homozygous wild type genotype presented higher 6-MMPN levels compared to those with the heterozygous genotype (p<0.0001).

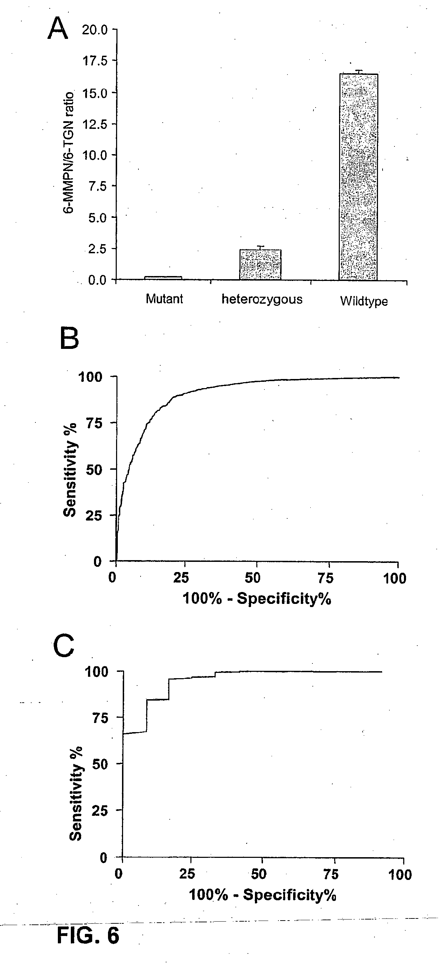

**Figure 6****:** illustrates 6-MMPN/6-TGN ratio and TPMT gene locus. (A) distribution of 6-MMPN/6-TGN metabolic ratio by TPMT genotype. Bars represent average ± SEM. (B) ROC curve analysis of homozygous wild type versus heterozygous with 6-MMPN/6-TGN ratio. (C) ROC curve analysis of heterozygous versus homozygous mutant with 6-MMPN/6-TGN ratio.

### DETAILED DESCRIPTION OF THE INVENTION

### I. DEFINITIONS

As used herein, the term "6-mercaptopurine drug" or "6-MP drug" refers to any drug that can be metabolized to an active 6-mercaptopurine metabolite that has therapeutic efficacy such as 6-TGN. Exemplary 6-mercaptopurine drugs as defined herein, include 6-mercaptopurine (6-MP) and azathioprine (AZA). As illustrated in FIG. 1, both 6-map and AZA can be metabolized to 6-mercaptopurine metabolites such as the exemplary 6-mercaptopurine metabolites shown in FIG. 1, including 6-thioguanine nucleotide (6-TGN), 6-methyl-mercaptopurine nucleotide (6-MMPN) and 6-thiouric acid. (Lennard, Eur. J. Clin. Pharmacol. 43:329-339 (1992)).

As used herein, the term "6-thioguanine nucleotide" or "6-TGN" refers to 6-thioguanine nucleotides or analogues thereof, including molecules having the same base structure, for example, 6-thioguanine ribonucleoside, 6-thioguanine ribonucleotide mono-, di- and tri-phosphate, 6-thioguanine deoxyribonucleoside and 6-thioguanine deoxyribonucleotide mono-, di-, and triphosphate. The term "6-TGN" also includes derivatives of 6-thioguanine, including chemical modifications of 6-TGN, so long as the structure of the 6-TG base is preserved.

As used herein, the term "6-methyl-mercaptopurine nucleotide" or "6-MMPN" refers to 6-methyl-mercaptopurine nucleotide or analogues thereof, including analogues having the same base structure, for example, 6-methyl-mercaptopurine ribonucleoside, 6-methyl-mercaptopurine ribonucleotide mono-, di-, and tri-phosphate, 6-methyl-mercaptopurine deoxyribonucleoside, and 6-methyl-mercaptopurine deoxyribonucleotide mono-, di- and tri-phosphate. The term "6-MMP" also includes derivatives of 6-methyl-mercaptopurine, including chemical modifications of 6-MMP.

As used herein, the term "drug" refers to a substance used as a medication or in the preparation of medication. According to the Food, Drug, and Cosmetic Act, a drug is a substance recognized in an official pharmacopoeia or formulary, a substance intended for use in the diagnosis, cure, mitigation, treatment, or prevention of disease, a substance other than food intended to affect the structure or function of the body, or a substance intended for use as a component of a medicine but not a device or a component, part, or accessory of a device.

As used herein, the term "drug providing 6-thioguanine nucleotide" refers to a drug that, upon administration to a subject, results in the production, manufacture, or release of the substance. In the present invention, such substances include, but are not limited to, 6-mercaptopurine, azathioprine, 6-thioguanine, and 6-methylmercaptopurine riboside.

As used herein, the term "subsequent dose" refers to a dose of a drug that is administered to a subject after a first dose of the same drug has already been administered to the same subject.

As used herein, the term "immune-mediated gastrointestinal disorder" refers to inflammatory bowel diseases (IBD) such as Crohn's disease and ulcerative colitis, lymphocytic colitis, microscopic colitis, collagenous colitis, autoimmune enteropathy, allergic gastrointestinal disease and eosinophilic gastrointestinal disease.

As used herein, the term "autoimmune disease" refers to a condition where the immune system attacks the cells, tissues, and organs of a subject's own body. Exemplary autoimmune diseases include, but are not limited to asthma, atherosclerosis, diabetic nephropathy, glomerulonephritis, inflammatory bowel disease, Crohn's disease, multiple sclerosis, pancreatitis, pulmonary fibrosis, psoriasis, restenosis, rheumatoid arthritis, or transplant rejection.

As used herein, the term "graft versus host disease" refers to a condition where cells from a non-host subject that are grafted into a host subject attack the host subject.

As used herein, the term "concentration level" refers to the concentration of a particular compound, drug or nucleotide in a subject. Concentration levels can be given in units of mol/L. One of skill in the art will appreciate that other units are also be appropriate.

As used herein, the term "predetermined toxicity level" refers to a specific toxic concentration level of 6-TGN wherein a specific toxic concentration level of 6-TGN is targeted. Preferred predetermined toxicity levels of 6-TGN include, but are not limited to, 350, 370, 390, 400, 410, 425, and 450 pmol per 8x10⁸ red blood cells. A predetermined toxicity level of 400 pmol per 8x10⁸ is especially relevant. One of skill in the art will appreciate that other predetermined toxicity levels are also useful in the present invention.

As used herein, the term "predetermined efficacy value" refers to a specific efficacious value of 6-TGN that is targeted. Preferred predetermined efficacy values of 6-TGN include, but are not limited to, 200, 210, 220, 230, 235, 240, 250, 260, 280, and 300 pmol per 8x10⁸ red blood cells. A predetermined efficacy value of 235 pmol per 8x10⁸ is especially relevant. One of skill in the art will appreciate that other predetermined efficacy values are also useful in the present invention.

As used herein, the term "ionizing source" refers to a method for ionizing a sample such as in a mass spectrometer. Exemplary methods for ionization include, but are not limited to fast-atom bombardment, electrospray ionization, secondary ion source, plasma desorption, matrix assisted laser desorption/ionization, atmospheric pressure ionization and field desorption.

As used herein, the term "mass-to-charge ratio" refers to the ratio of the mass of a detected fragment in a mass spectrometer over the charge of that same detected fragment. The mass-to-charge ratio is abbreviated as m/z.

"Recommend" or "recommending" as used herein refers to providing dosing instructions for a drug (*e*.*g*., a thiopurine) based on the active metabolite profile of the drug. Dosing instructions include, *e*.*g*., package inserts to accompany the drug, instructions or guidelines for using the drugs, lab results with preferred drug doses, data sheets, and look-up tables setting forth preferred drug doses.

As used herein, the term "analytical technique" refers to a method for determining a property of a particular substance. Preferred analytical techniques include those having an ionizing source, such as for example, mass spectrometry. One of skill in the art will appreciate that other analytical techniques can be used in the instant invention.

As used herein, the term "liquid chromatography tandem mass spectrometry" refers to mass spectrometry technique that is known to one of skill in the art and involves obtaining a sample from a liquid chromatograph system and coupling two stages of mass analysis so as to subject a particular fragment of the first ionization process to a subsequent ionization process.

As used herein, the term "6-MMP derivative" means 4-amino-5-(methythio)carbonyl imidazole.

As used herein, the term "6-TGMP" means 6-thioguanosine monophosphate.

As used herein, the term "6-TGDP" means 6-thioguanosine diphosphate.

As used herein, the term "6-TGTP" means 6-thioguanosine triphosphate.

As used herein, the term "Me6-TIMP" means ethyl 6-thioinosine monophosphate.

As used herein, the term "Me6-TIDP" means methyl 6-thioinosine diphosphate.

As used herein, the term "Me6-TITP" means methyl 6-thioinosine triphosphate.

As used herein, the term "TPMT" means thiopurine methyltransferase. TPMT genotyping is useful for predicting the effectiveness of 6-MP therapy in an IBD patient. Heterozygote patients are expected to have lower TPMT activity and should therefore be monitored for high levels of 6-TGN for possible toxic levels associated with leukopenia or bone marrow suppression. 6-MP drug doses can be reduced accordingly. Wild type homozygous patients are expected to have higher TPMT activity and should therefore be monitored to maintain an effective therapeutic level of 6-TG and to determine if patients develop toxic levels of 6-MMP. Homozygous patients deficient in TPMT activity can be treated with lower doses of a 6-MP drug provided that patients are closely monitored for toxicity such as leukopenia. Therefore, TPMT genotyping can be used to predict patient responsiveness to and potential toxicities associated with 6-MP drug therapy. Furthermore, TPMT genotyping can be combined with other methods of the invention to both determine TPMT genotype and to monitor 6-MP metabolites. TPMT genotyping can be particularly valuable when determining a starting dose of 6-MP drug therapy but can also be useful when adjusting 6-MP drug doses after therapy has begun

As used herein, the term "DTT" means dithiotreitol.

As used herein, the term "RSD" means relative standard deviation.

As used herein, the term "SEM" means standard deviation of the mean.

### II. METHODS

The present disclosure provides a method for optimizing therapeutic efficacy in a subject receiving a drug providing 6-thioguanine nucleotide. The method comprises: (a) determining a concentration level of 6-thioguanine nucleotide (6-TGN) in the subj ect using an analytical technique having an ionizing source; and (b) increasing the subsequent dose of the drug when the concentration level of 6-thioguanine nucleotide (6-TGN) is less than a predetermined efficacy value.

In certain aspects, when the methods herein require "increasing" or "decreasing" subsequent doses based on metabolite levels, the methods can in fact be "recommending" subsequent doses. In other words, once the metabolite concentration is determined, "increasing" or "decreasing" subsequent doses can be evidenced by inserts to accompany the concentration levels of metabolites, such as guidelines, data sheets, and look-up tables setting forth preferred drug doses.

Suitable ionizing sources include, but are not limited to, an electrospray ion source, an atmospheric pressure ionization source, and a matrix assisted laser desorption ion source. In certain aspects, the methods of the present disclosure use laser desorption ionization MS techniques. These techniques include, but are not limited to, MALDI, IR-MALDI, UV-MALDI, liquid-MALDI, surface-enhanced LDI (SELDI), surface enhanced neat desorption (SEND), desorption/ionization of silicon (DIOS), laser desorption / laser ionization MS, laser desorption/two-step laser ionization MS, and the like. Those of skill in the art will know of other ionization techniques as well as other mass spectrometric techniques useful in the present methods.

In other aspects, the methods of the present disclosure can also use electrospray ionization (ESI). In operation, mass spectrometry separates the ions according to their mass to charge ratio (m/z). Tandem mass spectrometers operate by using this separation of ions as a first fractionation step. Before entering the second mass spectrometer, ion fractions from the first are fragmented (e.g., collisionally dissociated by passage through a neutral gas, to induce fragmentation). These fragments exist as a family of subset ions of the original parent ions. Analysis of the m/z spectrum of these subset ions are used to determine the concentration level of the metabolite. In certain aspects, a transition of a mass-to-charge ratio (m/z) of 168→ 151 is useful to identify 6-TGN. The mass to charge ratio (m/z) of 158→110 transition is useful to identify 6-methyl-mercaptopurine nucleotide (6-MMPN). In certain aspects, the methods of the present invention further comprise liquid chromatography (LC) separation, such as the analytical technique, LC-tandem mass spectroscopy. In certain other aspects, single ion monitoring is the analytical technique.

In certain aspects, the methods of the present disclosure relate to optimizing therapeutic efficacy of 6-MP drug treatment of an immune-mediated GI disorder, including IBD such as Crohn's disease, or ulcerative colitis and subtypes thereof. The methods of the invention are particularly useful for treating patients dependent on steroid therapy for maintenance of remission.

As used herein, the phrase "optimizing therapeutic efficacy of 6-MP drug treatment" refers to adjusting the therapeutic dosage of a 6-MP drug such as 6-MP or azathioprine, so that the concentration of a 6-MP metabolite that is correlated with effective treatment is maintained. The methods of the invention allow the clinician to provide an individually optimized dosage of a 6-MP drug so as to achieve a target level of a 6-MP metabolite in a particular patient, thereby optimizing the effectiveness of 6-MP drug therapy in the patient.

As used herein, the phrase "determining a concentration level of 6-thioguanine nucleotide (6-TGNs)" in a subject refers to the amount or concentration level which can be conveniently assayed using for example red blood cells. In certain instances, as explained more fully below, the concentration level of 6-TGN is determined by measuring the amount or concentration of 6-thioguanine (6-TG).

As used herein, the phrase "determining a concentration level of 6-methylmercaptopurine nucleotide (6-MMPN)" in a subject refers to the amount or concentration level which can be conveniently assayed using for example red blood cells. In certain instances, as explained more fully below, the concentration level of 6-MMPN is determined by measuring the amount or concentration of a 6-MMP derivative such as 4-amino-5-(methythio)carbonyl imidazole.

Therapeutic efficacy generally is indicated by alleviation of one or more signs or symptoms associated with the disease. In the case of immune-mediated GI disorders, in particular IBD, therapeutic efficacy is indicated by alleviation of one or more signs or symptoms associated with the disease, including, for example, joint pain, arthritis, arthalgia, anorexia, growth failure, fistula closure, abdominal pain, diarrhea, recurrent fever, anemia, weight loss, rectal bleeding, inflammation of the intestine, and loose discharges of blood, pus and mucus. Other indications include, for example, a disease or disorder such as an immune-mediated gastrointestinal disorder, an autoimmune disease, and graft versus host disease.

In certain aspects, the predetermined efficacy value for 6-TGN is selected from the group of about 200, 210, 220, 230, 235, 240, 250, 260, 280, 300 and 350 pmol per 8x10⁸ red blood cells. Preferably, the predetermined efficacy value of 6-TGN is 235 pmol per 8x10⁸ red blood cells.

In certain aspects, increasing the subsequent dose of the drug when the concentration level of 6-thioguanine nucleotide (6-TGN) is less than a predetermined efficacy value can result in significant clinical improvement of the subject, as the 6-TGN levels increase. In other aspects, increasing the subsequent dose can have minimal changes in 6-TGN levels of the subject, but dramatic increases in 6-MMPN levels. This increase in 6-MMPN can have a dose dependant hepatotoxic event with this rise in 6-MMPN levels.

6-MP metabolite levels can be conveniently assayed using red blood cells because such cells are readily available from the patient, lack a nucleus and are easy to manipulate. However, it should be understood that any measurement that allows determination of 6-MP metabolite levels can be used. For example, leukocytes can be used to measure 6-MP metabolite levels, which can be correlated with 6-MP metabolite levels in erythrocytes. Regardless of the method employed to measure 6-MP metabolites, one skilled in the art can readily measure 6-MP metabolite levels in a sample, for example, in leukocytes or DNA obtained from a patient, and correlate the level of 6-MP metabolites to the values disclosed herein, which were determined using RBC.

In certain aspects, the methods of the present disclosure use a rapid sample treatment procedure where erythrocyte proteins are precipitated using perchloric acid in presence of DTT (Erdmann, G. R. et al., Biomed. Claromatogr. 4:47-51 (1990)) to reduce disulfide crosslinks of thiol-containing nucleotides with that of sulfhydryl residues on proteins. The resulting acidic extract provides the protons necessary to hydrolyze the covalent base-ribose bond with formation of 6-TG from 6-TGNs and of 4-amino 5-(methyl)thiocarbonyl imidazole from 6-MMPNs. Both analytes can then be subsequently detected using LC MS/MS. In certain aspects, the chromatographic separation is performed on a reverse phase dC 18 column having improved retention capability for polar 6-TGN. In certain preferred embodiments, positive electrospray ionization with a triple quadrupole mass spectrometer operating in multiple reaction-monitoring (MRM) mode is used to quantitate each metabolite, with high collision energy to enhance specificity for 6-TGN over endogenous co-eluting polar compounds. Other advantages of the MS/MS detection compared to the conventional HPLC-UV is an improved throughput as DTT does not interfere with the resolution.

It is important to note that the kinetics of conversion of TGNs (TGMP, TGDP, TGTP) to 6-TG was slightly faster than the conversion of 6-MMPNs (MeTIMP, MeTIDP, MeTITP) to the 6-MMP derivative (**Figure 3**). This is probably because the later process requires the additional conversion of neo-formed 6-MMP (from 6-MMPN) into the derivative. Thus, a complete conversion of 6-MMP to the derivative during the sample treatment procedure ensures a complete hydrolysis of TGNs to 6-TG. Thus, the simultaneous determination of 6-TG and 6-MMP derivative after hydrolysis appear mandatory using 6-TG and 6-MMP standards as the complete conversion of 6-MMP to its derivative is the internal control for the conversion of TGN to 6-TG.

In another embodiment, the present disclosure provides a method for reducing toxicity in a subject receiving a drug providing 6-thioguanine nucleotide (6-TGN), comprising: (a) determining a concentration level of 6-thioguanine nucleotide (6-TGN) in the subject using an analytical technique having an ionizing source; and (b) decreasing the subsequent dose of the drug when the concentration level of 6-thioguanine nucleotide is greater than a predetermined toxicity value.

As disclosed herein, the level of a 6-MP metabolite can be determined in a subject treated with a 6-MP drug and compared to a predetermined toxic level of a 6-MP metabolite such as 6-TGN or 6-MMPN to adjust future 6-MP drug administration, thereby reducing toxicity in the subject. For example, as disclosed herein, levels of 6-TGN above the predetermined toxicity value indicates that a patient is likely to experience toxicity, in particular hematologic toxicity such as leukopenia. Accordingly, a level of 6-TGN above about 400 pmol per 8x10⁸RBC can be a predetermined toxic level of 6-TGN, which indicates that the amount of 6-MP drug subsequently administered should be decreased. Suitable predetermined toxicity values of 6-TGN can be selected from the group consisting of about 350, 370, 390, 400, 410, 425, and 450 pmol per 8x10⁸ red blood cells. A relevant predetermined toxicity value of 6-TGN is 400 pmol per 8x10⁸ red blood cells.

It is further understood that, when a patient is determined to have a level of a 6-MP metabolite such as 6-TGN or 6-MMPN higher than a predetermined toxic level, one skilled in the art can make a determination as to whether a subsequent dose of 6-MP drug should be decreased. For example, if the level of a 6-MP metabolite such as 6-TGN or 6-MMPN is higher than a predetermined toxic level, one skilled in the art can monitor for toxic side effects by measuring one or more of the toxicities associated with 6-MP drug treatment, as disclosed herein. A level of 6-TGN greater than the predetermined toxicity value is associated with increased risk of leukopenia or bone marrow suppression. Therefore, one skilled in the art can measure white blood cells (WBC) in a patient having levels of 6-TGN higher than a predetermined toxic level to determine if the patient is exhibiting signs of reduced WBC counts. If such a patient exhibits signs of leukopenia or bone marrow suppression, the 6-MP drug dose can be reduced. However, if it is determined that a patient has levels of a 6-MP metabolite higher than a predetermined toxic level, but does not exhibit signs of leukopenia or other 6-MP drug toxicities, one skilled in the art can determine that the current 6-MP drug dose can be maintained. Based on measuring 6-MP metabolite levels and determining signs or symptoms of toxicities associated with 6-MP drug treatment, one skilled in the art can determine whether a 6-MP drug dose should be maintained or decreased. As such, a level of a 6-MP metabolite higher than a predetermined toxic level can indicate a need to measure a toxicity associated with 6-MP drug treatment such as measuring WBCs or any of the other signs or symptoms of toxicities associated with 6-MP drug treatment to determine if the 6-MP drug dose should be adjusted.

The present disclosure provides a method for reducing toxicity in a subject receiving a drug providing 6-thioguanine nucleotide, comprising: (a) determining a concentration level of 6-methyl-mercaptopurine nucleotide (6-MMPN) in the subject using an analytical technique having an ionizing source; and (b) decreasing the subsequent dose of the drug when the concentration level of 6-methyl-mercaptopurine nucleotide (6-MMPN) is greater than 6-methyl-mercaptopurine toxicity value.

A useful 6-MP metabolite for predicting the likelihood of toxicity is 6-methyl-mercaptopurine nucleotide (6-MMP). The level of 6-MMPN can be used to predict toxicity in a patient treated with a 6-MP drug. The predetermined 6-MMPN toxicity value is selected from the group of about 5500, 5600, 5700, 6000, 6500, 7000, 7500, and 8000 pmol per 8x10⁸ red blood cells. An especially relevant predetermined 6-MMPN toxicity value is about 5700 pmol per 8x10⁸ red blood cells. The predetermined toxic levels of 6-MMPN disclosed herein are useful for treating immune-mediated GI disorders, using IBD, as well as non-IBD autoimmune diseases. According to a method of the disclosure if the level of 6-MMPN is above a predetermined toxic level, the subsequent administration of a 6-MP drug can be decreased to minimize toxicity.

In one embodiment, the present invention provides a method for optimizing therapeutic efficacy for treatment of an immune-mediated gastrointestinal disorder, comprising: determining a concentration level using an analytical technique having an ionizing source of 6-thioguanine nucleotide (6-TGNs) or 6-methyl-mercaptopurine nucleotide (6-MMPN) in a subject administered a drug providing 6-thioguanine nucleotide, wherein the subject has an immune-mediated gastrointestinal disorder, wherein a concentration level of 6-thioguanine nucleotide (6-TGN) less than a predetermined efficacy value indicates a need to increase the amount of the drug subsequently administered to the subject, and wherein a concentration level of 6-thioguanine nucleotide (6-TGN) greater than a predetermined toxicity level, or a concentration level of 6-methyl-mercaptopurine nucleotide (6-MMPN) greater than a predetermined 6-MMPN toxicity level, indicates a need to decrease the amount of the drug subsequently administered to the subject.

The present disclosure provides a method for determining the TPMT genotype of a subject administered a drug providing 6-thioguanine nucleotide, comprising: (a) determining a concentration level of 6-thioguanine nucleotide (6-TGN) in the subject; (b) determining a concentration level of 6-methyl-mercaptopurine (6-MMPN) in the subject; and (c) calculating the ratio of 6-MMPN/6-TGN concentration levels to determine the TPMT genotype of the subject. A ratio of about 0.1 to about 0.5 (e.g., 0.2) indicates a homozygous mutant genotype, whereas a ratio of about 2.0 to about 3.0 (e.g., 2.4) indicates a heterozygous genotype. A ratio of about 16.0 to about 17.0 (e.g., 16.6) indicates a wild type genotype.

In certain other aspects, a ratio greater than 2.385 indicates a wild type genotype, whereas, when the ratio has the following value: 0.295<ratio<2.385, the predicted genotype is heterozygous. When the ratio is less than 0.295, the predicted genotype is homozygous mutant.

The present disclosure provides a method for optimizing therapeutic efficacy or reducing toxicity in a subject receiving a drug providing 6-thioguanine nucleotide, comprising: (a) determining a concentration level of 6-thioguanine nucleotide (6-TGN) in the subject using an analytical technique having an ionizing source; and (b) recommending a subsequent dose of the drug when the concentration level of 6-thioguanine nucleotide is compared to a predetermined value. The predermined value is a predetermined efficacy value, a predetermined toxicity value or both. Predetermined efficacy values include 200, 210, 220, 230, 235, 240, 250, 260, 280, 300 and 350 pmol per 8x10⁸ red blood cells, wherein a more preferred value is 235 pmol per 8x10⁸ red blood cells. Predetermined toxicity values include 350, 370, 390, 400, 410, 425, and 450 pmol per 8x10⁸ red blood cells, wherein a preferred predetermined toxicity value is 400 pmol per 8x10⁸ red blood cells. In certain aspects, a laboratory provides dosing instructions for the drug such as a package insert to accompany the drug, a guideline for using the drug, a lab result with preferred drug doses, a data sheet, or a look-up table setting forth preferred drug doses.

Additional embodiments of the disclosure relate to the communication of assay results, the concentration levels of the metabolites, the subsequent doses, or diagnoses to technicians, physicians, patients, hospitals and the like. In certain embodiments, computers will be used to communicate the information, results, or doses, to the interested parties, e.g., physicians and their patients. In some embodiments, the methods will be performed or the results analyzed in a country or jurisdiction which differs from the country or jurisdiction to which the results or diagnoses are communcated.

The concentration levels of the metabolites, the subsequent doses, or diagnoses can be communicated to the subject by the subject's treating physician. Alternatively, the concentration levels or subsequent doses can be sent to a subject by email or communicated to the subject by phone. A computer can be used to communicate the concentration levels or subsequent doses by email or phone. In certain embodiments, the message containing concentration levels or subsequent doses, or diagnostic test can be generated and delivered automatically to the subject using a combination of computer hardware and software which will be familiar to artisans skilled in telecommunications. In certain embodiments, all or some of the method steps, including the determination of concentration levels of the metabolites, subsequent doses, diagnoses, assaying of samples, and communicating the results can be carried out in diverse (e.g., foreign) jurisdictions.

As such, the present disclosure provides a method for communicating to an individual a subsequent dose of a drug providing 6-thioguanine nucleotide .in a subject, comprising: (a) determining a concentration level of 6-thioguanine nucleotide (6-TGN) in the subject using an analytical technique having an ionizing source; and (b) communicating to an individual the subsequent dose via a computer-generated medium, such as over the internet.

### III. EXAMPLES

### A. METHODS

### 1. Chemicals and preparation of standards

6-thioguanine (6-TG), 6-methylmercaptopurine (6-MMP) and dithiotreitol (DTT) were purchased from SIGMA Laboratories (St. Louis, MO, USA). 6-thioguanosine monophosphate (6-TGMP), 6-thioguanosine diphosphate (6-TGDF), 6-thioguanosine triphosphate (6-TGTP), methyl 6-thioinosine monophosphate (Me6-TIMP), methyl 6-thioinosine diphosphate (Me6-TIDP) and methyl 6-thioinosine triphosphate (Me6-TITP) were purchased from Jena Biosciences (Jena, Germany). All thiopurine nucleotides were sodium salts and at a final concentration of 10mM. 4-Amino-5-(methylthio) carboxy-imidazole (6-MMP derivative) was prepared using the following procedure (Zimm, S. et al., Anal. Biochem. 160:1-6 (1987)): 6-MMP standard was diluted to a final concentration of 1mM in mol/L hydrochloric acid and heated for 60 minutes at 100°C. Under these conditions a total conversion of 6-MMP to its derivative was complete (not shown). HPLC grade acetonitrile was purchased from Fisher Chemicals (Pittsburgh, PA, USA), perchloric acid (700mL/L) was obtained from Merck.

6-TG and 6-MMP were prepared by dissolution in 50mM sodium hydroxide. The above standards were diluted to a final concentration of 800 and 8000 µM in water and stored at -70°C. The stock solutions were stable for at least 12 months under these conditions. Standard curves were prepared by adding known amounts of 6TG and 6-MMP to a pool of red blood cell (RBC) hemolysates isolated from healthy blood donors (Blood Bank, San Diego, CA). The quantification of thiopurine nucleotides was performed using an equimolar mixture of 6-TGMP, 6-TGDP and 6-TGTP for 6-TGN, and of Me6-TIMP,Me6-TIDP and Me6-TITP for 6-MMPN. Standard curves ranged from 0.25 to 20 µM for 6-TG and from 2.5 to 200 µM for 6-MMP. 6-TG and 6-MMP derivative signals were fitted by quadratic regression using peak area versus 1/x weighted concentrations.

### 2. Treatment procedure of erythrocyte hemolysate

A volume of 100 µl of RBC hemolysate (~10⁹ RBCs) was homogenized with 300 µl of water containing 0.2M DTT in a 1.5-ml polypropylene tube. A 40 µl 700 mL/L perchloric acid was added, and the mixture was immediately thoroughly vortexed for 10 seconds (to precipitate proteins) and centrifuged for 10 minutes. A 250 µl of acidic supernatant was subsequently transferred in a 12x32 mm glass vial containing a 6x31 mm glass insert, capped, and heated at 100°C in a dry block heater (15 mm wells) for 60 minutes (otherwise indicated). After cooling, a 20 µl aliquot was directly injected onto the LC/MS/MS system.

### 3. Liquid chromatography electrospray tandem mass spectrometry system

The liquid chromatograph was an Agilent 1100 HPLC system consisting of a binary pump, a system controller, and an auto-injector. The chromatographic system was connected to an API 2000 (Applied Biosystem, Forster City, CA) with an internal diverter (Valco valve). The chromatographic separation was performed on a 2.1cm x 150 mm Atlantis dCl8 (Waters, Milford, MA, USA), 3µm particle size, protected by an Atlantis dC18 10mm guard column. The mobile phase A consisted of ammonium acetate (2.5 mM) with formic acid at a final concentration of 0.1 %. Mobile phase B consisted of acetonitrile containing 0.1 % formic acid.

The separation of 6-TG and 6-MMP derivative was achieved with a 8 minute isocratic elution with 95% mobile phase A and 5% mobile phase B at a final flow rate of 0.3 ml/min. Eluents were directed to the waste for the first 2.0 minutes and introduced into the turbo-ion spray source thereafter. Ionization was achieved in the positive-ion mode with an ionization voltage of 5500 V. The heater probe was set at 400 °C. Orifice plate potentials consisted of a declustering potential of 10V, a focusing potential of 400V and an entrance potential of 12V. Sample analysis was performed in the multiple-reaction monitoring (MRM) mode using an m/z 168→151 transition for 6-TG and an m/z 158→110 transition for 6-MMP derivative. Collision energy was set at 40V with nitrogen as the collision gas. One sample was injected every 8 minutes. Integration of peak areas and determination of the concentrations were performed with the Analyst 1.4 software.

### 4. Precision, accuracy and recoveries

Intra- and inter-day precision and accuracy were determined by analyzing low, medium and high concentrations of 6-TG and 6-MMP added to RBC hemolysates. Intra-day analysis was performed with 10 spiked replicates, whereas inter-day evaluation was assessed with on 10 different days. Accuracy was calculated as the percentage difference between the measured concentrations from each spiked samples relative to the target concentration (measured concentration/target concentration x 100%). Precision was defined by estimating the coefficient of variation (CV in %). Recoveries were determined by comparing the peak height of 6-TG and 6-MMP spiked to RBC to samples prepared at the same concentration in water. The kinetics of conversion of thiopurine nucleoside monophosphate (6-TGMP; Me6-TIMP), diphosphate (6-TGDP; Me6-TIDP) and triphosphate (6-TGTP; Me6-TITP) to 6-TG and 6-MMP derivative was determined using nucleotide standards spiked to RBC hemolysate at a final concentration of 10 µmol/L for 6-thioguanine nucleotides and of 100 µmol/L for methyl 6-mercaptopurine nucleotides.

### 5. Application to patients receiving azathioprine and mercaptopurine and method comparison

EDTA whole blood from patients having received azathioprine and mercaptopurine and shipped to our remote location for the routine monitoring of thiopurine therapy were used to compare the method using the HPLC-UV method (Erdmann, G. R. et al., Biomed. Chromatogr. 4:47-51 (1990)) with that of the LC/MS/MS assay. After a 5-minute centrifugation step to separate plasma and buffy coat from RBCs, the RBCs were washed 2 times with 2 volumes of saline. Erythrocytes were counted before freezing to normalize metabolite levels. Packed RBCs were stored at -70°C until analysis. 6-TGN and 6-MMPN measured using different analytical methods were compared using linear regression (coefficient of correlation, slope and intercept). Results are expressed as pmol/8x10⁸ RBCs or as µM/L packed RBCs as appropriate.

### 6. Application at the Thiopurine Methyltransferase (TPMT) Gene locus

RBC thiopurine levels determined concomitantly with TPMT genotype in our clinical laboratory were extracted from our database. To protect patient privacy, all identifiers linking patient individual data to our laboratory results were removed. Common polymorphisms affecting TPMT activity (G238C, G460A, A719G) were measured as described previously using a PCR-RFLP procedure (Yates, C.R. et al., Ann. Intern. Med. 126:608-614 (1997)). TPMT*2 allele consists of the presence of the 238C polymorphism, TPMT*3A allele consists of the presence of both 460A and 719G polymorphisms and TPMT*3C allele consists of the presence of the 719G polymorphism. Group comparison was determined using the Kruskal-Wallis ANOVA and χ² tests as appropriate, and relative risks were calculated with 95% CI.

### B. RESULTS

### 1. LC/MS/MS assay of RBC thiopurine nucleotides

Chromatogram of blank RBCs versus RBCs spiked with 6-TG and 6-MMP and subjected to the sample treatment procedure is illustrated in **Figure 2**. Regression correlation coefficients were >0.995 for 6-TG and 6-MMP derivative. Typical equations describing the standard curves were: 6-TG; y=0.96x² +620x +6.6; 6-MMP derivative, y= 0.39x² +968x +6.6 (y: peak area; x: spiked concentration). Intra-day and inter-day precision and accuracy of the assay highlight the precision of our method (Table I).

The limit of detection, defined as three times the signal-to-noise ratio, was 0.1 µmol/L for 6-TG and 0.5 µM for 6-MMP derivative. The conversion of thioguanine and methyl-mercaptopurine nucleosides mono, di, and triphosphate to 6-TG and 6-MMP derivative was complete after a 60-minute acid hydrolysis at 100°C at a concentration of 10 µmol/L 6-TGN (~1000 pmol/8x10⁸ RBCs) and of 100 µmol/L 6-MMPN (~10,000 pmol/8x10⁸ RBCs) (**Figure 3**). Before acid hydrolysis, no 6-TG and 6-MMP derivative was detected in RBC spiked with 6-thioguanine and 6-methylmercaptopurine nucleotides whereas during hydrolysis, 6-TG and 6-MMP derivative peaks appeared (not shown). Complete conversion of 6-TGN and 6-MMPN, was demonstrated with an accuracy ranging from 90% to 110% and a coefficient of variation < 10%.

**Table I. Precision and accuracy for 6-TG and 6-MMP in RBCs.**

| The intra-day data summarizes 10 different replicates at each concentration for each compound (6-TG and 6-MMP) in one experiment. The inter-day data summarizes ten different experiments from ten consecutive days with three replicates each day and at each concentration for each compound. | | | | | | | |
|---|---|---|---|---|---|---|---|
| *Abbreviations*: RSD.: Relative standard deviation of the mean. | | | | | | | |
| | | Intra-day (n=10) | | | Inter-day (n=10) | | |
| Spiked Standard | Target concentration µmol/L | Mean observed concentration µmol/L | RSD % | Mean accuracy of target value (%) | Mean observed concentration µmol/L | RSD % | Mean accuracy of target value (%) |
| 6-TG | 10.00 | 9.62 | 3.6 | 96.3 | 10.10 | 3.6 | 100.7 |
| | 2.50 | 2.47 | 6.7 | 99.0 | 2.49 | 4.8 | 99.7 |
| | 0.50 | 0.49 | 6.0 | 99.9 | 0.49 | 3.6 | 99.9 |
| | | | | | | | |
| 6-MMP | 100.0 | 100.1 | 3.3 | 100.1 | 99.4 | 3.0 | 99.4 |
| | 25.0 | 25.1 | 4.1 | 100.3 | 25.3 | 2.9 | 101.2 |
| | 5.0 | 4.9 | 3.5 | 98.3 | 5.0 | 3.5 | 100.2 |

**Table II. Precision and accuracy for 6-TGN and 6-MMPN standards in RBCs after acid hydrolysis.**

| The data summarizes 4 to 5 experiment from 4 to 5 different days with 3 replicates each day at each concentration for each equimolar mixture of thiopurine nucleoside mono-, di- and triphosphate. | | | | | |
|---|---|---|---|---|---|
| *Abbreviations*: RSD.: Relative standard deviation of the mean. | | | | | |
| Spiked Standard | Target concentration µmol/L | Measured after hydrolysis | Mean observed concentration µmol/L | RSD % | Mean accuracy of target value (%) |
| 6-TGN: | 10.00 | 6-TG | 9.19 | 4.5 | 91.9 |
| 6-TGMP+6-TGDP | 2.50 | | 2.48 | 7.1 | 99.2 |
| +6-TGTP | 0.50 | | 0.51 | 9.7 | 102.4 |
| | | | | | |
| 6-MMPN: | 100.0 | 6-MAP | 98.5 | 7.1 | 98.5 |
| Me6-TIMP+Me6-TIDP | 25.0 | derivative | 26.0 | 5.1 | 103.9 |
| +Me6-TITP | 5.0 | | 5.3 | 5.5 | 106.1 |

### 2. Method comparison

### In 50 RBC samples from patients under azathioprine and mercaptopurine therapy

there was no difference between 6-TGN and 6-MMPN concentrations measured following the LC/MS/MS method (6-TGN: 2.70±1.64 µmol/L; 6-MMPN 47.6+54.9 µmol/L; n=50) and the 6-TGN and 6-MMPN measured using the previously validated HPLC/UV assay (6-TGN: 3.03±1.80 µmol/L; 6-MMPN 52.6±58.7 µmol/L; n=50). This was evidenced by a slope of 1.09 (intercept 0.08; coefficient of correlation: 0.98) for TGN and of 1.07 (intercept 1.7; coefficient of correlation: 0.99) for 6-MMPNs between the two methods (**Figure 4**). The LC/MS/MS method was also equivalent to the HPLC-UV method (Erdmann, G. R. et al., Biomed. Chromatogr. 4:47-51 (1990)) that uses similar sample treatment procedure.

### 3. Application at the Thiopurine methyltransferase locus

In 1523 individuals, the distribution of TPMT genotypes consisted of 1385 patients (91%) carrier of the homozygous wild-type genotype (TPMT*1/*1), 132 patients (9%) carriers of the heterozygous genotype (TPMT*1/*3A, n=100; TPMT*1/*2, n=5; TPMT*1/*3C, n=27) and 6 patients carriers of the homozygous mutant genotype (TPMT*3A/*3A, n=3; TPMT*3A/*3C, n=2; TPMT*2/*3C, n=1).

Average TGN was 231±5 (SEM) pmol/8x10⁸ RBCs (median 195, n=1523) and average 6-MMPN was 2506±99 (SEM) pmol/8x10⁸ RBCs (median 1116, n=1523). Patient carriers of the heterozygous genotype presented higher 6-TGN concentrations compared to those carriers of the homozygous wild type genotype (408±23 vs 202±3 pmol/8x10⁸ RBCs; p<0.0001). In contrast those carriers of the homozygous wild type genotype presented higher 6-MMPN levels compared to those with the heterozygous genotype (2679±107 vs 808±144 pmol/8x10⁸ RBCs; p<0.0001). 6-MMPN was not detected in patient carriers of the homozygous mutant genotype whereas 6-TGN concentrations reached levels above 1000 pmol/8x10⁸ RBCs (average 2681±406 pmol/8x10⁸ RBCs. (Figure 5). A total of 46% patients presented 6-TGN levels below 235 pmol/8x10⁸ RBCs, 9% presented 6-TGN above 400 pmol/8x10⁸ RBCs, and 24% presented 6-TGN levels in the therapeutic range 235-400 pmol/8x10⁸ RBCs. Only 12% of patients presented 6-MMPN levels above 5700 pmol/8x10⁸ RBCs.

The distribution of TGN and 6-MMPN levels by TPMT genotype is presented in **Table III.** All patients with the homozygous mutant-genotype presented 6-TGN levels in the range associated with leukopenia (>400 pmol/8x10⁸ RBCs) with no 6-MMPN detected. Heterozygosity for TPMT was associated with a lower risk for 6-TGN<235 pmol/8x10⁸ RBCs (relative risk 0.18; p<0.0001) but a higher risk for 6-TGN above 400 pmol/8x10⁸ RBCs (relative risk 10.8; p<0.0001) compared to patients with the wild type genotype. TGN levels within the therapeutic range (235<TGN<400 pmol/8x10⁸ RBCs) was achieved in 25.7% patients with the wild type genotype and 33.8% of patients with the heterozygous genotype. Carriers of the wild-type genotype had a 4.4 higher risk (CI 95%: 1.6-11.2; p<0.0001) of reaching 6-MMPN levels above 5700 pmol/8x10⁸ RBCs compared to those with the heterozygous genotype.

**Table III. Therapeutic thresholds of RBC 6-TGN and 6-MMPN by TPMT genotype**

| RBC thiopurine nucleotides levels <235 pmol/8x10e⁸ RBCs are associated with a greater likelihood of treatment failure whereas TGN levels above 400 pmol/8x10e⁸ RBCs are associated with higher risk of leukopenia. 6-MMPN levels>5700 pmol/8x10e⁸ RBCs are associated with increased risk for hepatotoxicity. Percentage patient with number is given with relative risk for heterozygous versus wild type. | | | | | |
|---|---|---|---|---|---|
| | TPMT homozygous Wild type n=1385 | TPMT heterozygous n=132 | TPMT homozygous mutant n=6 | Relative risk heterozygous vs. homozygous wild type (CI 95%) | p level |
| 6-TGN<235 | 69.4% (961) | 12.8% (17) | 0% (0) | 0.18 (0.12-0.29) | <0.0001 |
| pmol/8x10⁸ RBCs | | | | | |
| | | | | | |
| 6-TGN>400 | 4.9% (68) | 53.0% (70) | 100% (6) | 10.8 (8.1-14.3) | <0.0001 |
| pmol/8x10⁸ RBCs | | | | | |
| | | | | | |
| 6-MMPN>5700 | 13.2% (183) | 3.0% (4) | 0% (0) | 0.22 (0.09-0.61) | <0.0001 |
| pmol/8x10⁸ RBCs | | | | | |

### 4. TPMT gene locus and methyl-thiopurine metabolic ratio

Patients carrier of the homozygous mutant genotype had lower 6-MMPN/6-TGN ratio (average 0.20±0.06) than those with the heterozygous genotype (average 2.37±0.35; p<0.001). Conversely, those with the wild type genotype has higher 6-MMPN/6-TGN ratio (average 16.55±0.30) than those with the heterozygous genotype (p<0.001) (**Figure 6A**). ROC curves established that a metabolite ratio above 2.385 predicted (ROC area=0.908) the homozygous wild type versus heterozygous genotype with a 83.4% (CI 95: 82.5.6-84.3 %) sensitivity and 84.0% (CI 95: 81.0-86.7 %) specificity (**Figure 6B**). A metabolite ratio below 0.295 predicted the homozygous mutant versus heterozygous genotype (ROC area= 0.908) with a sensitivity and specificity of 83.3% (CI 95: 51.6-97.9 %) and 95.8% (CI 95: 93.9-97.1 %) respectively (p<0.0001) (**Figure 6C**). Finally, the concordance between molecular TPMT genotype and TPMT genotype inferred using the 6-MMPN/6-TGN ratio cutoffs at 2.385 and 0.295 is presented in Table IV and illustrate the capability of the phenotypic variable to predict genotype.

Patients carriers of the homozygous wild type genotype presented a increased risk of having TGNs levels below the therapeutic thresholds (235 pmol/8x10⁸ cells) compared to those with the heterozygous genotype, while those with the heterozygous genotype presented higher risk of having TGNs above 400pmol/8x10⁸ cells (leukopenia risk). These data also illustrate the dilemma facing physicians. Patients carriers of the wild type genotype are at higher risk of elevated hepatotoxic 6-MMPNs levels upon dose escalation and have increased risk for treatment failure than those with the heterozygous genotype (Dubinsky, M.C. et al., Gastroenterology 122:904-915 (2002); Oh, K. T. et al., Rheumatology (Oxford) 43:156-163 (2004); Marra, C. A. et al. J Rheumatol 29:2507-2512 (2002)). Conversely, those with the heterozygous genotype would be less likely to experience hepatotoxicity due to 6-MMPN formation, but area at higher risk of myelosuppression compared to those with the wild type genotype. This illustrates the need to determine TPMT genotype prior to therapy and to carefully adjust dosage in these patients to avoid potentially fatal extensive accumulation of TGNs.

**Table IV. RBC methyl-thiopurine metabolic ratio cutoffs and concordance to TPMT genotype**

| The table summarizes observed TPMT genotypes compared to predicted genotype using 6-TGN/6-MMPN ratio cutoffs of 2.385 and | | | | |
|---|---|---|---|---|
| 0.295. Percentage of patients (number) categorized using the metabolic ratio compared to the true genotype is given. | | | | |
| | | | TPMT genotype | |
| | | Wild type (n=6490) | Heterozygous (n=682) | Mutant (n=12) |
| 6-MMPN/6-TGN | Predicted | | | |
| Ratio>2.385 | Wild type | 83.4% (5415) | 16.5% (1072) | <0.05% (3) |
| 0.295<Ratio<2.385 | Heterozygous | 16.0% (109) | 79.8% (544) | 4.3% (29) |
| Ratio<0.295 | Mutant | 0%(0) | 15.7% (2) | 83.3% (10) |

## Claims

1. A method for optimizing therapeutic efficacy in a subject receiving a drug providing 6-thioguanine nucleotide (6-TGN), said method comprising:
(a) determining a concentration level of 6-TGN in said subject using an analytical technique having an ionizing source, wherein said analytical technique is a mass spectrometer;
(b) determining a concentration level of 6-methyl-mercaptopurine nucleotide (6-MMPN) in said subject using an analytical technique having an ionizing source, wherein said analytical technique is multiple reaction monitoring using a mass spectrometer, wherein determination of 6-TGN and 6-MMPN concentration levels occur simultaneously; and
(c) recommending increasing the subsequent dose of said drug when said concentration level of 6-TGN is less than a predetermined 6-TGN efficacy value; or
(d) recommending decreasing the subsequent dose of said drug when said concentration level of 6-MMPN is greater than a predetermined 6-MMPN toxicity value.

2. A method for reducing toxicity in a subject receiving a drug providing 6-thioguanine nucleotide (6-TGN), said method comprising:
(a) determining a concentration level of 6-TGN in said subject using an analytical technique having an ionizing source;
(b) determining a concentration level of 6-methyl-mercaptopurine nucleotide (6-MMPN) in said subject using an analytical technique having an ionizing source, wherein said analytical technique is multiple reaction monitoring using a mass spectrometer, wherein determination of 6-TGN and 6-MMPN concentration levels occur simultaneously; and
(c) recommending decreasing the subsequent dose of said drug when said concentration level of 6-TGN is greater than a predetermined 6-TGN toxicity value; or
(d) recommending decreasing the subsequent dose of said drug when said concentration level of 6-MMPN is greater than a predetermined 6-MMPN toxicity value

3. A method for optimizing therapeutic efficacy for treatment of an immune-mediated gastrointestinal disorder, said method comprising:
determining a concentration level using an analytical technique having an ionizing source wherein said analytical technique is multiple reaction monitoring using a mass spectrometer, of 6-thioguanine nucleotide (6-TGN) and 6-methyl-mercaptopurine nucleotide (6-MMPN) in a subject administered a drug providing 6-TGN, said subject having said immune-mediated gastrointestinal disorder, wherein determination of 6-TGN and 6-MMPN concentration levels occur simultaneously,
wherein a concentration level of 6-TGN less than a predetermined 6-TGN efficacy value indicates recommending an increase in the amount of said drug subsequently administered to said subject, and
wherein a concentration level of 6-TGN greater than a predetermined 6-TGN toxicity value, or a concentration level of 6-MMPN greater than a predetermined 6-MMPN toxicity value indicates recommending a decrease in the amount of said drug subsequently administered to said subject.

4. The method of any one of claims 1 to 2, wherein said subject has a disease or disorder selected from the group consisting of an immune-mediated gastrointestinal disorder, an autoimmune disease, and graft versus host disease.

5. The method of any one of claims 1 to 4, wherein said drug is selected from the group consisting of 6-mercaptopurine, azathioprine, 6-thioguanine, and 6-methylmercaptopurine riboside.

6. The method of any one of claims 1 to 5, wherein said ionizing source is selected from a group consisting of an electrospray ion source, an atmospheric pressure ionization source, and a matrix assisted laser desorption ion source.

7. The method of any one of claims 1 to 6, wherein said analytical technique further comprises liquid chromatography (LC) separation.

8. The method of claim 7, wherein said concentration level of 6-thioguanine nucleotide (6-TGN) or 6-methyl-mercaptopurine nucleotide (6-MMPN) is determined using LC-tandem mass spectroscopy.

9. The method of any one of claims 1, 2 and 3 to 8, wherein a transition of a mass-to-charge ratio (m/z) of 168→151 is used to identify 6-TGN.

10. The method of any one of claims 1-9, wherein the mass to charge ratio (m/z) of 158→110 transition is used to identify 6-MMPN.

11. The method of any one of claims 1, 3-12 wherein said predetermined 6-TGN efficacy value is selected from the group consisting of 200, 210, 220, 230, 235, 240, 250, 260, 280, 300 and 350 pmol per 8x10⁸ red blood cells.

12. The method of claim 11, wherein said predetermined 6-TGN efficacy value is 235 pmol per 8x10⁸ red blood cells.

13. The method of any one of claims 2-12, wherein said predetermined 6-TGN toxicity value is selected from the group consisting of 350, 370, 390, 400, 410, 425, and 450 pmol per 8x10⁸ red blood cells.

14. The method of claim 13, wherein said predetermined 6-TGN toxicity value is 400 pmol per 8x10⁸ red blood cells.

15. The method of any one of claims 1-14, wherein said predetermined 6-MMPN toxicity value is selected from the group consisting of 5500, 5600, 5700, 6000, 6500, 7000, 7500, and 8000 pmol per 8x10⁸ red blood cells.

16. The method of claim 15, wherein said predetermined 6-MMPN toxicity value is 5700 pmol per 8x10⁸ red blood cells.

17. The method of any one of claims 1-16, where a testing laboratory provides dosing instructions for said drug selected from the group consisting of a package insert to accompany said drug, a guideline for using said drug, a lab results with preferred drug doses, a data sheet, and a look-up table setting forth preferred drug doses.

## Patentansprüche

1. Verfahren zur Optimierung der therapeutischen Wirksamkeit bei einem Patienten, der ein 6-Thioguanin-Nukleotid (6-TGN) lieferndes Medikament erhält, wobei das Verfahren Folgendes umfasst:
(a) Bestimmen eines Konzentrationsgehalts von 6-TGN in dem Patienten unter Einsatz einer analytischen Methode mit einer Ionisierungsquelle, wobei die analytische Methode aus einem Massenspektrometer besteht;
(b) Bestimmen eines Konzentrationsgehalts von 6-Methyl-Mercaptopurin-Nukleotid (6-MMPN) in dem Patienten unter Einsatz einer analytischen Methode mit einer Ionisierungsquelle, wobei die analysierende Methode eine multiple Reaktionsüberwachung unter Verwendung eines Massenspektrometers ist, wobei die Bestimmung von Konzentrationsgehalten von 6-TGN und 6-MMPN gleichzeitig erfolgt; und
(c) Empfehlen einer Erhöhung der nachfolgenden Dosis des Medikaments, wenn der Konzentrationsgehalt von 6-TGN unter einem vorgegebenen 6-TGN Wirksamkeitswert liegt; oder
(d) Empfehlen einer Verringerung der nachfolgenden Dosis des Medikaments, wenn der Konzentrationsgehalt von 6-MMPN über einem vorgegebenen 6-MMPN Toxizitätswert liegt.

2. Verfahren zur Verringerung der Toxizität bei einem Patienten, der ein 6-Thioguanin-Nukleotid (6-TGN) lieferndes Medikament erhält, wobei das Verfahren Folgendes umfasst:
(a) Bestimmen eines Konzentrationsgehalts von 6-TGN in dem Patienten unter Einsatz einer analytischen Methode mit einer Ionisierungsquelle;
(b) Bestimmen eines Konzentrationsgehalts von 6-Methyl-Mercaptopurin-Nukleotid (6-MMPN) in dem Patienten unter Einsatz einer analytischen Methode mit einer Ionisierungsquelle, wobei die analytische Methode eine multiple Reaktionsüberwachung unter Verwendung eines Massenspektrometers ist, wobei die Bestimmung von Konzentrationsgehalten von 6-TGN und 6-MMPN gleichzeitig erfolgt; und
(c) Empfehlen einer Verringerung der nachfolgenden Dosis des Medikaments, wenn der Konzentrationsgehalt von 6-TGN höher ist als ein vorgegebener 6-TGN Toxizitätswert; oder
(d) Empfehlen einer Verringerung der nachfolgenden Dosis des Medikaments, wenn der Konzentrationsgehalt von 6-MMPN höher ist als ein vorgegebener 6-MMPN Toxizitätswert.

3. Verfahren zur Optimierung der therapeutischen Wirksamkeit bei Behandlung einer immunvermittelten Magendarmerkrankung, wobei das Verfahren Folgendes umfasst:
Bestimmen, unter Einsatz einer analytischen Methode mit einer Ionisierungsquelle, wobei die analytische Methode eine multiple Reaktionsüberwachung unter Verwendung eines Massenspektrometers darstellt, eines Konzentrationsgehalts von 6-Thioguanin-Nukleotid (6-TGN) und 6-Methyl-Mercaptopurin-Nukleotid (6-MMPN) in einem Patienten, dem ein 6-TGN lieferndes Medikament verabreicht wird, wobei der Patient an der immunvermittelten Magendarmerkrankung leidet, wobei die Bestimmung von Konzentrationsgehalten von 6-TGN und 6-MMPN gleichzeitig erfolgt,
wobei ein Konzentrationsgehalt von 6-TGN unter einem vorgegebenen Wirksamkeitswert von 6-TGN die Empfehlung einer Erhöhung der Menge des dem Patienten nachfolgend verabreichten Medikaments indiziert, und
wobei ein Konzentrationsgehalt von 6-TGN über einem vorgegebenen Toxizitätswert von 6-TGN oder ein Konzentrationsgehalt von 6-MMPN über einem vorgegebenen Toxizitätswert von 6-MMPN die Empfehlung einer Verringerung der Menge des dem Patienten nachfolgend verabreichten Medikaments indiziert.

4. Verfahren gemäß einem der Ansprüche 1 oder 2, wobei der Patient an einer Krankheit oder Störung ausgewählt aus der Gruppe bestehend aus einer immunvermittelten Magendarmerkrankung, einer Autoimmunkrankheit sowie einer Transplantat-Wirt-Reaktion leidet.

5. Verfahren gemäß einem der Ansprüche 1 bis 4, wobei das Medikament aus der Gruppe bestehend aus 6-Mercaptopurin, Azathioprin, 6-Thioguanin und 6-Methylmercaptopurin-Nukleotid-Ribosid ausgewählt ist.

6. Verfahren gemäß einem der Ansprüche 1 bis 5, wobei die Ionisierungsquelle aus einer Gruppe bestehend aus einer Elektrospray-Ionenquelle, einer Atmosphärendruck-Ionisierungsquelle sowie einer matrixunterstützten Laserdesorptions-Ionenquelle ausgewählt ist.

7. Verfahren gemäß einem der Ansprüche 1 bis 6, wobei die analytische Methode weiterhin eine Abscheidung durch Flüssigkeitschromatographie (LC) umfasst.

8. Verfahren gemäß Anspruch 7, wobei der Konzentrationsgehalt von 6-Thioguanin-Nukleotid (6-TGN) oder 6-Methyl-Mercaptopurin-Nukleotid (6-MMPN) unter Verwendung von LC- Massenspektroskopie in Tandemanordnung bestimmt wird.

9. Verfahren gemäß einem der Ansprüche 1, 2 und 3 bis 8, wobei ein Übergang einer spezifischen Ladung (m/z) von 168→151 zur Ermittlung von 6-TGN verwendet wird.

10. Verfahren gemäß einem der Ansprüche 1 bis 9, wobei der Übergang der spezifischen Ladung (m/z) von 158→110 zur Ermittlung von 6-MMPN verwendet wird.

11. Verfahren gemäß einem der Ansprüche 1, 3 bis 12, wobei der vorgegebene Wirksamkeitswert von 6-TGN aus der Gruppe bestehend aus 200, 210, 220, 230, 235, 240, 250, 260, 280, 300 und 350 pmol pro 8x10⁸ rote Blutkörperchen ausgewählt ist.

12. Verfahren gemäß Anspruch 11, wobei der vorgegebene Wirksamkeitswert von 6-TGN 235 pmol pro 8x10⁸ rote Blutkörperchen beträgt.

13. Verfahren gemäß einem der Ansprüche 2 bis 12, wobei der vorgegebene Toxizitätswert von 6-TGN aus der Gruppe bestehend aus 350, 370, 390, 400, 410, 425 und 450 pmol pro 8x10⁸ rote Blutkörperchen ausgewählt ist.

14. Verfahren gemäß Anspruch 13, wobei der vorgegebene Toxizitätswert von 6-TGN 400 pmol pro 8x10⁸ rote Blutkörperchen beträgt.

15. Verfahren gemäß einem der Ansprüche 1 bis 14, wobei der vorgegebene Toxizitätswert von 6-MMPN aus der Gruppe bestehend aus 5500, 5600, 5700, 6000, 6500, 7000, 7500 und 8000 pmol pro 8x10⁸ rote Blutkörperchen ausgewählt ist.

16. Verfahren gemäß Anspruch 15, wobei der vorgegebene Toxizitätswert von 6-MMPN 5700 pmol pro 8x10⁸ rote Blutkörperchen beträgt.

17. Verfahren gemäß einem der Ansprüche 1 bis 16, wobei ein Versuchslabor Dosieranleitungen für das Medikament ausgewählt aus der Gruppe bestehend aus einem dem Medikament beiliegenden Beipackzettel, einer Richtlinie zur Anwendung des Medikaments, Laborergebnissen mit bevorzugten Dosierungen des Medikaments, einem Datenblatt sowie einer Nachschlageliste mit bevorzugten Dosierungen des Medikaments bereitstellt.

## Revendications

1. Procédé pour optimiser l'efficacité thérapeutique chez un sujet recevant un médicament fournissant un nucléotide 6-thioguanine (6-TGN), ledit procédé comprenant :
(a) détermination d'un niveau de concentration de 6-TGN chez ledit sujet en utilisant une technique analytique ayant une source d'ionisation, dans lequel ladite technique analytique est un spectromètre de masse ;
(b) détermination d'un niveau de concentration de nucléotide de 6-méthylmercaptopurine (6-MMPN) chez ledit sujet en utilisant une technique analytique ayant une source d'ionisation, dans lequel ladite technique analytique est une surveillance de réactions multiples utilisant un spectromètre de masse, dans lequel la détermination des niveaux de concentration de 6-TGN et 6-MMPN a lieu simultanément ; et
(c) recommandation d'une augmentation de la dose ultérieure dudit médicament lorsque ledit niveau de concentration de 6-TGN est inférieur à une valeur prédéterminée d'efficacité de 6-TGN ; ou
(d) recommandation d'une diminution de la dose ultérieure dudit médicament lorsque ledit niveau de concentration de 6-MMPN est supérieur à une valeur prédéterminée de toxicité de 6-MMPN.

2. Procédé pour réduire la toxicité chez un sujet recevant un médicament fournissant un nucléotide 6-thioguanine (6-TGN), ledit procédé comprenant :
(a) détermination d'un niveau de concentration de 6-TGN chez ledit sujet en utilisant une technique analytique ayant une source d'ionisation ;
(b) détermination d'un niveau de concentration de nucléotide de 6-méthylmercaptopurine (6-MMPN) chez ledit sujet en utilisant une technique analytique ayant une source d'ionisation, dans lequel ladite technique analytique est une surveillance de réactions multiples utilisant un spectromètre de masse, dans lequel la détermination des niveaux de concentration de 6-TGN et 6-MMPN a lieu simultanément ; et
(c) recommandation d'une diminution de la dose ultérieure dudit médicament lorsque ledit niveau de concentration de 6-TGN est supérieur à une valeur prédéterminée de toxicité de 6-TGN ; ou
(d) recommandation d'une diminution de la dose ultérieure dudit médicament lorsque ledit niveau de concentration de 6-MMPN est supérieur à une valeur prédéterminée de toxicité de 6-MMPN.

3. Procédé pour optimiser l'efficacité thérapeutique pour le traitement d'un trouble gastro-intestinal à médiation immunitaire, ledit procédé comprenant :
détermination d'un niveau de concentration, en utilisant une technique analytique ayant une source d'ionisation dans lequel ladite technique analytique est une surveillance de réactions multiples utilisant un spectromètre de masse, de nucléotide 6-thioguanine (6-TGN) et de nucléotide de 6-méthyl-mercaptopurine (6-MMPN) chez un sujet à qui est administré un médicament fournissant du 6-TGN, ledit sujet ayant ledit trouble gastro-intestinal à médiation immunitaire, dans lequel la détermination des niveaux de concentration de 6-TGN et 6-MMPN a lieu simultanément,
dans lequel un niveau de concentration de 6-TGN inférieur à une valeur prédéterminée d'efficacité de 6-TGN indique une recommandation d'une augmentation de la quantité dudit médicament administré ultérieurement audit sujet,
et
dans lequel un niveau de concentration de 6-TGN supérieur à une valeur prédéterminée de toxicité de 6-TGN, ou un niveau de concentration de 6-MMPN supérieur à une valeur prédéterminée de toxicité de 6-MMPN indique une recommandation de diminution de la quantité dudit médicament administré ultérieurement audit sujet.

4. Procédé selon l'une quelconque des revendications 1 ou 2, dans lequel ledit sujet présente une maladie ou un trouble choisi parmi le groupe constitué d'un trouble gastro-intestinal à médiation immunitaire, d'une maladie auto-immune, et de la maladie du greffon contre l'hôte.

5. Procédé selon l'une quelconque des revendications 1 à 4, dans lequel ledit médicament est choisi parmi le groupe constitué de 6-mercaptopurine, azathioprine, 6-thioguanine, et 6-méthylmercaptopurine riboside.

6. Procédé selon l'une quelconque des revendications 1 à 5, dans lequel ladite source d'ionisation est choisie parmi le groupe constitué d'une source d'ions à électronébulisation, une source d'ionisation à pression atmosphérique, et une source d'ions à désorption laser par matrice.

7. Procédé selon l'une quelconque des revendications 1 à 6, dans lequel ladite technique analytique comprend en outre une séparation par chromatographie liquide (LC).

8. Procédé selon la revendication 7, dans lequel ledit niveau de concentration de nucléotide 6-thioguanine (6-TGN) ou de nucléotide de 6-méthyl-mercaptopurine (6-MMPN) est déterminé en utilisant une spectrométrie de masse en tandem LC.

9. Procédé selon l'une quelconque des revendications 1, 2 et 3 à 8, dans lequel une transition d'un rapport masse sur charge (m/z) de 168→151 est utilisée pour identifier le 6-TGN.

10. Procédé selon l'une quelconque des revendications 1-9, dans lequel la transition du rapport masse sur charge (m/z) de 158→ 110 est utilisée pour identifier le 6-MMPN.

11. Procédé selon l'une quelconque des revendications 1, 3-12, dans lequel ladite valeur prédéterminée d'efficacité de 6-TGN est choisie parmi le groupe constitué par 200, 210, 220, 230, 235, 240, 250, 260, 280, 300 et 350 pmol par 8x10⁸ de globules rouges.

12. Procédé selon la revendication 11, dans lequel ladite valeur prédéterminée d'efficacité de 6-TGN est 235 pmol par 8x10⁸ de globules rouges.

13. Procédé selon l'une quelconque des revendications 2-12, dans lequel ladite valeur prédéterminée de toxicité de 6-TGN est choisie parmi le groupe constitué par 350, 370, 390, 400, 410, 425, et 450 pmol par 8x10⁸ de globules rouges.

14. Procédé selon la revendication 13, dans lequel ladite valeur prédéterminée de toxicité de 6-TGN est 400 pmol par 8x10⁸ de globules rouges.

15. Procédé selon l'une quelconque des revendications 1-14, dans lequel ladite valeur prédéterminée de toxicité de 6-MMPN est choisie parmi le groupe constitué par 5500, 5600, 5700, 6000, 6500, 7000, 7500, et 8000 pmol par 8x10⁸ de globules rouges.

16. Procédé selon la revendication 15, dans lequel ladite valeur prédéterminée de toxicité de 6-MMPN est 5700 pmol par 8x10⁸ de globules rouges.

17. Procédé selon l'une quelconque des revendications 1-16, dans lequel un laboratoire d'essai fournit des instructions de dosage pour ledit médicament choisi parmi le groupe constitué par une notice destinée à accompagner ledit médicament, une directive pour utiliser ledit médicament, des résultats de laboratoire avec des doses préférées de médicament, une fiche de données, et une table de correspondance énonçant des doses préférées de médicament.
